(19) Europäisches Patentamt
European Patent Office
Office européen des brevets

(11) Veröffentlichungsnummer: **0 300 324 B1**

(12) **EUROPÄISCHE PATENTSCHRIFT**

(45) Veröffentlichungstag der Patentschrift: **22.07.92**

(51) Int. Cl.$^5$: **C07D 231/12**, C07D 233/22

(21) Anmeldenummer: **88111080.3**

(22) Anmeldetag: **12.07.88**

Die Akte enthält technische Angaben, die nach dem Eingang der Anmeldung eingereicht wurden und die nicht in dieser Patentschrift enthalten sind.

(54) **Verfahren zur Herstellung von acylierten Imidazolen und Pyrazolen.**

(30) Priorität: **21.07.87 DE 3724035**

(43) Veröffentlichungstag der Anmeldung:
**25.01.89 Patentblatt 89/04**

(45) Bekanntmachung des Hinweises auf die Patenterteilung:
**22.07.92 Patentblatt 92/30**

(84) Benannte Vertragsstaaten:
**AT BE CH DE FR GB IT LI NL**

(56) Entgegenhaltungen:

**CHEMICAL ABSTRACTS, Band 107, Nr. 13, 28. September 1987, Columbus, Ohio, US; REITER, L.A.: "Synthesis of 4(5)-acyl-1-substituted 5-acyl- and 1-substituted 4-acyl-1H-imidazoles from 4-aminoisoxazoles." Seite 624, Spalte 1, Zusammenfassung-Nr. 115 533w**

**CHEMICAL ABSTRACTS, Band 105, Nr. 9, 1. September 1986, Columbus, Ohio, US; REITER, L.A.: "A general synthesis of 4(5)-acylimidazoles from 4-acyl-aminoisoxazoles." Seite 642, Spalte 2, Zusammenfassung-Nr. 78 874u**

**K. Schofield, Heteroaromatic Nitrogen Compounds, The Azoles, Cambridge University Press, 1976**

(73) Patentinhaber: **BASF Aktiengesellschaft**
**Carl-Bosch-Strasse 38**
**W-6700 Ludwigshafen(DE)**

(72) Erfinder: **Lermer, Helmut, Dr.**
**D 3,4**
**W-6800 Mannheim 1(DE)**
Erfinder: **Hoelderich, Wolfgang, Dr.**
**Mannheimer Strasse 18 c**
**W-6710 Frankenthal(DE)**
Erfinder: **Dockner, Toni, Dr.**
**Grossgasse 6**
**W-6701 Meckenheim(DE)**
Erfinder: **Koehler, Hermann, Dr.**
**Roentgenstrasse 7**
**W-6711 Beindersheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Anmeldung betrifft ein Verfahren zur Herstellung von acylierten Imidazolen und Pyrazolen durch Direktacylierung von entsprechenden unsubstituierten oder substituierten Imidazolen und Pyrazolen mit Acylierungsmitteln in Gegenwart von Katalysatoren.

Direkte C-Acylierungen von Imidazolen in Friedel-Crafts-Reaktionen waren nicht bekannt bzw. wurden für technisch nicht ausführbar gehalten (A.R. Katritzky, C.W. Rees, Comprehensive Heterocyclic Chemistry, Vol. 5, S. 402 (1984), Pergamon Press; K. Hofmann. The Chemistry of Heterocyclic Compounds, Vol. 6: Imidazole and its derivatives, Part I, S. 49 u. S. 59 (1953), Interscience Publishers).

Daher war man gezwungen, auf andere Möglichkeiten der Darstellung auszuweichen, z.B. die photochemische Umlagerung von N-Acetylimidazolen (J.L. La Mattina et al., J. Org. Chem. 48, 897-8 (1983), oder die Umsetzung von 4-Formylimidazolen mit einem Grignard-Reagens und nachfolgende Oxidation (z.B.: R. Paul et al., J. Med. Chem. 28, 1704-16 (1985), oder die Hydrogenolyse eines 4-Acylamino-5-methylisoxazols (EP 156 644, Pfizer).

Diese Methoden sind jedoch aufwendig und im technischen Maßstab nicht durchführbar.

Auch der Pyrazol-Kern ist einer Friedel-Crafts-Acylierung nicht zugänglich. Acylpyrazole werden daher nach aufwendigen Verfahren hergestellt, z.B. durch Umsetzung von Pyrazol-3-carbonylchlorid mit Benzol (Friedel-Crafts-Reaktion) oder durch Synthese des Pyrazol-Kerns aus entsprechend substituierten acyclischen Vorstufen (The Chemistry of Heterocyclic Compounds, Vol. 20, R.H. Wiley (ed.), S. 117 - 124 (1967)-).

Die technisch interessante Direktacylierung am Pyrazol-Kern ist bisher noch nicht beschrieben worden.

Es wurde nun gefunden, daß man direkt C-acylierte Imidazole und Pyrazole der Formeln (I) und (II)

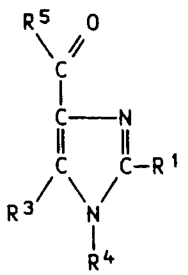
(I)

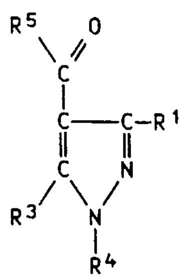
(II)

in denen $R^1$ und $R^3$ gleich oder verschieden sein können und unabhängig voneinander H-, Alkyl- mit 1 bis 12 Kohlenstoffatomen, Alkenyl-mit bis zu 12 Kohlenstoffatomen, Alkoxyl- mit 1 bis 12 Kohlenstoffatomen, Aryl-, Aralkyl- oder Alkylarylreste,
$R^4$ = H-, Alkyl mit 1 bis 12 Kohlenstoffatomen, Alkenyl- mit bis zu 12 Kohlenstoffatomen, Aryl-, Aralkyl- oder Alkylarylreste,
$R^5$ = Alkyl- mit 1 bis 12 Kohlenstoffatomen, Alkenyl- mit bis zu 12 Kohlenstoffatomen, Aryl-, Aralkyl-, Alkylaryl- oder Carboxyreste bedeuten,
aus Imidazolen bzw. Pyrazolen der Formeln (III) bzw. (IV)

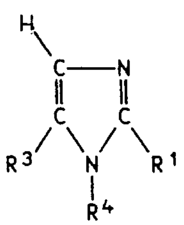
(III)

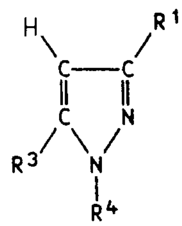
(IV)

in denen $R^1$, $R^3$ und $R^4$ die oben genannten Bedeutungen haben, mit Acylierungsmitteln der Formel (V)

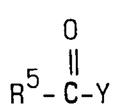
(V)

in der $R^5$ die obengenannte Bedeutung hat und Y = Halogenid-, Alkoxy-, Acyloxy-, Hydroxy-, Amid- oder Carboxyreste bedeutet, in Gegenwart von zeolithischen Katalysatoren herstellt.

Die Umsetzung von 2-Methylimidazol mit Essigsäureanhydrid zu 4-Acetyl-2-methylimidazol an einem Zeolith-Katalysator läßt sich beispielsweise durch folgende Formelgleichung wiedergeben

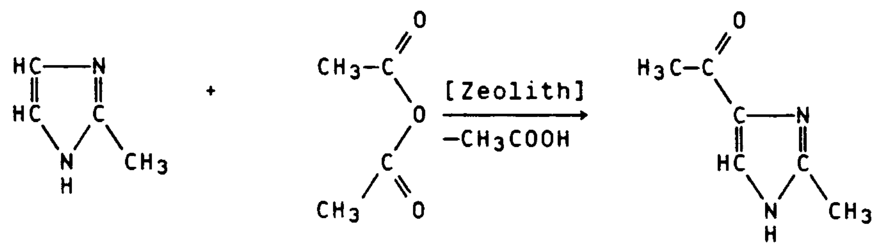


Die analoge Umsetzung von 4-Methylimidazol liefert in der Hauptsache 5-Acetyl-4-methylimidazol, daneben aber auch 2-Acetyl-4-methylimidazol.

Die Nachteile der bekannten mehrstufigen Herstellungsweise werden durch das erfindungsgemäße Verfahren elegant vermieden.

Für das erfindungsgemäße Verfahren geeignete Heteroaromaten sind Imidazole und Pyrazole der oben angegebenen Formeln (III) bzw. (IV), z.B. Imidazol, 2-Methylimidazol, 1-Methylimidazol, 4-Methylimidazol, 2-Ethyl-4-methylimidazol, 4-n-Butylimidazol, 2-Isopropylimidazol, 2-Isopropyl-4-ethylimidazol, 2,4-Di-n-butylimidazol, 2-Phenyl-, 4-Phenylimidazol, 4-Benzylimidazol, Pyrazol, 3-Methylpyrazol, 3-n-Butylpyrazol, 3-Isopropylpyrazol, 4-Phenylpyrazol und 4-Benzylpyrazol.

Als Acylierungsmittel für das erfindungsgemäße Verfahren kann man gängige Verbindungen z.B. Essigsäurechlorid, Essigsäure, Essigsäureanhydrid, Essigsäuremethylester, Essigsäureethylester, Essigsäureamid, Propionsäurechlorid, Propionsäure, Propionsäureanhydrid, Buttersäure, Buttersäurechlorid, Buttersäureanhydrid, Isobuttersäure, Isobuttersäurechlorid, Isobuttersäureanhydrid, Crotonsäure, Crotonsäureanhydrid, Isoprensäure, Valeriansäure, Valeriansäurechlorid, Capronsäure, Capronsäureester, Pivalinsäure, Pivalinsäurechlorid, Acrylsäure, Acrylsäuremethylester, Methacrylsäure, Methacrylsäureester, Penten-3-säuremethylester, Penten-2-säuremethylester, Penten-4-säuremethylester, Sorbinsäure, Sorbinsäuremethylester, Benzoesäure, Benzosäurechlorid, Benzosäuremethylester, o-, m-, p-Fluorbenzoesäure, o-, m-, p-Fluorbenzoesäurechlorid, o-, m-, p-Chlorbenzoesäure, o-, m-, p-Methylbenzoesäure, o-, m-, p-Methylbenzoesäurechlorid, o-, m-, p-Methoxybenzoesäure, o-, m-, p-Methoxybenzoesäure chlorid, o-, m-, p-Isopropylbenzoesäure, o-, m-, p-Isopropylbenzoesäurechlorid, 2,3-Dimethyl-, 2,3-Difluor-, 2,3-Dichlor-, 2,3-Dimethoxybenzoesäure bzw. -benzoesäurechlorid, Phenylessigsäure, Phenylessigsäurechlorid, Phenylessigsäureanhydrid, Zimtsäure, Zimtsäurechlorid, Malonsäure, Malonsäureanhydrid, Phthalsäure, Phthalsäureanhydrid, Terephthalsäure und Terephthalsäurechlorid verwenden.

Als Katalysatoren werden bei dem erfindungsgemäßen Verfahren Zeolithe eingesetzt. Zeolithe sind kristalline Aluminosilikate, die eine hochgeordnete Struktur mit einem starren dreidimensionalen Netzwerk von $SiO_4$- oder $AlO_4$-Tetraedern besitzen, die durch gemeinsame Sauerstoffatome verbunden sind. Das Verhältnis der Si- und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekeln besetzt.

In den Zeolithen können anstelle von Aluminium auch andere Elemente wie B, Ga, Fe, Cr, V, As, Sb oder Be in das Gitter eingebaut werden, oder das Silicium kann durch ein vierwertiges Element wie Ge, Ti, Zr, Hf ersetzt werden.

Entsprechend ihrer Struktur werden die Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeeder, wodurch unterschiedlich große Hohlräume und Poren entstehen, unterscheidet man Zeolithe vom Typ A, L, X oder Y.

Unter den für das erfindungsgemäße Verfahren in Betracht kommenden Katalysatoren sind z.B. die Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasit-Typs, d.h. dealuminierte Zeolithe. Verfahren zur Herstellung solcher Zeolithe sind beschrieben in "Catalysis by Zeolites" Band 5 aus Studies in Surface Science and Catalysis,

ed. B. Imelik et al., Elsevier Scientific Publishing Comp. 1980, S. 203 und "Crystal Structures of Ultra-stable Faujasites" Advances in Chemistry Series Nr. 101, American Chemical Society Washington, DC, S. 226 ff (1971) und in US-PS 4 512 961.

Vorteilhaft verwendet man Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet, sowie durch Porengrößen, die zwischen denen der Zeolithe vom Typ A und denen vom Typ X oder Y liegen.

Die verwendbaren Zeolithe können unterschiedliche chemische Zusammensetzung aufweisen, geeignet sind, z.B. Alumino-, Boro-, Eisen-, Beryllium-, Gallium-, Chrom-, Arsen-, Antimon- und Wismutsilikatzeolithe oder deren Gemische sowie Alumino-, Boro-, Gallium-, Eisengermanatzeolithe oder deren Gemische. Insbesondere eignen sich die Alumino-, Boro- oder Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin-oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727. Die erhaltenen Aluminosilikatzeolithe enthalten je nach Wahl der Ausgangsstoffmengen ein $SiO_2/Al_2O_3$-Verhältnis von 10 bis 40 000. Derartige Aluminosilikatzeolithe kann man auch in etherischem Medium wie Diethylenglykoldimethylether, in alkoholischem Medium wie Methanol oder 1,4-Butandiol oder in Wasser synthetisieren.

Der Borosilikatzeolith wird z.B. bei 90 bis 200 °C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch isotaktische Borosilikatzeolithe, hergestellt nach EP 34 727 kann man vorteilhaft verwenden. Man kann auch Borosilikatzeolithe verwenden, die statt aus wäßriger Aminlösung aus einer etherischen Lösung, z.B. Diethylenglykoldimethylether oder aus alkoholischer Lösung, z.B. 1,6-Hexandiol auskristallisiert werden.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Durck.

Zu den verwendbaren silicumreichen Zeolithen ($SiO_2/Al_2O_3$ größer oder gleich 10) gehören auch die bekannten ZSM-Typen sowie Ferrierit und NU-1 sowie Silicalite®, ein Molekularsieb, ein sog. Silica Polymorph.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90 : 10 bis 40 : 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, $TiO_2$, Gemische aus hochdispersem $SiO_2$ und hochdispersem $Al_2O_3$ sowie Ton. Nach der Verformung werden die Extrudate oder Preßlinge bei 110 °C/16 h getrocknet und bei 500 °C/16 h calciniert.

Man erhält auch gut geeignete Katalysatoren, wenn der isolierte Alumino-bzw. Borosilikatzeolith direkt nach der Trocknung verformt wird und erst nach der Verformung einer Calcinierung unterworfen wird. Die Alumino- und Borosilikatzeolithe können in reiner Form, ohne Binder, als Stränge oder Tabletten eingesetzt werden, wobei als Verstrangungs- oder Peptisierungsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden können.

Liegt der Zeolith aufgrund der Art seiner Herstellung nicht in der katalytisch aktiven, aciden H-Form vor, sondern in der Na-Form, dann kann diese durch Ionenaustausch, z.B. mit Ammoniumionen, und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abtrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht z.B. darin,

daß man den unverformten oder verformten Zeolithen mit Metallsalzen durch einen Ionenaustausch oder durch Imprägnierung dotiert. Als Metalle werden Alkalimetalle, wie Li, K, Cs, Erdalkalimetalle, wie Mg, Ca, Ba, Erdmetalle, wie B, Al, Ga, Übergangsmetalle, wie Cu, Zn, Cr, Mn, Fe, Co, Ni, W und Mo, Edelmetalle, wie Pd, Pt, Rh, Ir und Seltene Erdmetalle, wie Ce, La, Pr, Nd, eingesetzt.

Zweckmäßigerweise führt man die Dotierung so durch, daß man den verformten Zeolithen in einem Steigrohr vorlegt und bei 20 bis 100 °C eine wäßrige oder ammoniakalische Lösung eines Halogenids oder eines Nitrats der voranbeschriebenen Metalle überleitet. Ein derartiger Ionenaustausch kann an der Wasserstoff-, Ammonium-, Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2$ x 3 $H_2O$ oder $Co(NO_3)_2$ x 6 $H_2O$ oder $Ce(NO_3)_3$ x 6 $H_2O$ oder $La(NO_3)_3$ x 6 $H_2O$ oder $Cs_2CO_3$ in Wasser löst. Mit dieser Lösung wird der verformte oder unverformte Zeolith eine gewisse Zeit, etwa 30 Minuten, getränkt. Die eventuell überstehende Lösung wird am Rotationsverdampfer von Wasser befreit. Danach wird der getränkte Zeolith bei etwa 150 °C getrocknet und bei 550 °C calciniert. Dieser Tränkvorgang kann mehrmals hintereinander vorgenommen werden, um den gewünschten Metallgehalt einzustellen.

Es ist auch möglich, z.B. eine wäßrige $Co(NO_3)_2$-Lösung oder ammoniakalische $Pd(NO_3)_2$-Lösung herzustellen und darin den reinen pulverförmigen Zeolithen bei 40 bis 100 °C unter Rühren etwa 24 h aufzuschlämmen. Nach Abfiltrieren, Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber eine wäßrige $Co(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert.

Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft so vor, daß man Zeolithe in Pulverform mit 1 n Phosphorsäure 1 h bei 80 °C behandelt. Nach der Behandlung wird mit Wasser gewaschen, bei 110 °C/16 h getrocknet und bei 500 °C/20 h calciniert. Nach einer anderen Arbeitsweise behandelt man die Zeolithe vor oder nach ihrer Verformung mit Bindemitteln, z.B. 1 bis 3 h bei Temperaturen von 60 bis 80 °C mit einer 3- bis 25 gew.%igen, insbesondere 12- bis 20 Gew.%igen wäßrigen Salzsäure. Anschließend wird der so behandelte Zeolith mit Wasser gewaschen, getrocknet und bei 400 bis 500 °C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von 0,5 bis 5 h, vorzugsweise 1 bis 3 h. Nach Isolierung, z.B. durch Abfiltrieren und Auswaschen, des zeolithischen Materials wird dieses zweckmäßig bei Temperaturen von 100 °C bis 160 °C, getrocknet und bei Temperaturen von 450 °C bis 600 °C calciniert. Gemäß einer weiteren bevorzugten Ausführungsform für die Säurebehandlung wird das zeolithische Material nach seiner Verformung mit Bindemittel bei erhöhter Temperatur, zweckmäßig bei Temperaturen von 50 °C bis 90 °C, vorzugsweise 1 bis 3 h mit Salzsäure, im allgemeinen mit 3 - 25 gew.%iger Salzsäure, vorzugsweise mit 12 - 20 gew.%iger Salzsäure, behandelt. Anschließend wird das zeolithische Material im allgemeinen ausgewaschen und zweckmäßig bei Temperaturen von 100 bis 160 °C getrocknet und bei Temperaturen von 450 °C bis 600 °C calciniert. Auch eine aufeinanderfolgende Behandlung mit HF und HCl ist gegebenenfalls vorteilhaft.

Nach einer anderen Arbeitsweise kann man Zeolithe durch Aufbringen von Phosphorverbindungen, wie Trimethylphosphat, Trimethoxyphosphin, primärem, sekundärem oder tertiärem Natriumphosphat modifizieren. Besonders vorteilhaft hat sich die Behandlung mit primären Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelgut

eingesetzt werden.

Die für die erfindungsgemäße Umwandlung in der Regel gewählten Reaktionsbedingungen liegen bei der bevorzugten Gasphasenreaktion bei 300 bis 600 ° C, vorzugsweise 400 bis 550 ° C, und einer Belastung WHSV von 0,1 bis 20 $h^{-1}$, bevorzugt 0,5 bis 5 $h^{-1}$ (g Einsatzgemisch/g Katalysator und Stunde). Die Reaktion läßt sich in einem Festbett oder Wirbelbett ausführen.

Es ist aber auch möglich, die Reaktion in der Flüssigphase in Suspensions-, Riesel- oder Sumpffahrweise bei Temperaturen zwischen 50 und 300 ° C, bevorzugt zwischen 100 und 250 ° C und einer Belastung g Ausgangsstoff : g Katalysator = 100 : 1 bis 5 : 1, bevorzugt 60 : 1 bis 10 : 1 durchzuführen.

Das Verfahren wird in der Regel bei Normaldruck oder je nach Flüchtigkeit der Ausgangsverbindung bei vermindertem Druck oder erhöhtem Druck durchgeführt. Die Durchführung erfolgt vorzugsweise kontinuierlich.

Schwerflüchtige oder feste Ausgangsstoffe werden in gelöster Form, z.B. in THF-, Toluol- oder Petrolether-Lösung, eingesetzt. Generell ist eine Verdünnung des Ausgangsstoffes mit derartigen Lösungsmitteln oder mit Inertgasen, wie $N_2$, Ar, He oder mit $H_2O$-Dampf möglich.

Nach der Umsetzung werden die entstandenen acylierten Imidazole oder Pyrazole durch übliche Verfahren, z.B. durch Destillation aus dem Reaktionsgemisch isoliert; nicht umgesetzte Ausgangsstoffe werden gegebenenfalls in die Umsetzung zurückgeführt.

Die folgenden Beispiele veranschaulichen die Erfindung.

Beispiel 1 - 14

Die Reaktionen in der Gasphase werden unter isothermen Bedingungen in einem Rohrreaktor (2,5 cm Innendurchmesser, 80 cm Länge f. Beispiel 1 und 2); Wendel, 0,6 cm Innendurchmesser, 90 cm Länge f. Beispiele 3 bis 14) mindestens 6 h lang durchgeführt. Die Reaktionsprodukte werden durch übliche Methoden abgetrennt und durch GC/MS, NMR und Bestimmung des Schmelzpunkts charakterisiert. Die quantitative Bestimmung der Reaktionsprodukte und der Ausgangsprodukte erfolgt gaschromatographisch oder durch HPLC (Hochleistungsflüssigchromatographie).

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50 : 50 Gew.%) bei 170 ° C unter autogenem Druck in einem Rührautoklaven hergestellt. Nach Abfiltrieren und Auswaschen wird das kristalline Reaktionsprodukt bei 100 ° C/24 h getrocknet und bei 500 ° C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit einem Verformungshilfsmittel 2-mm-Stränge hergestellt, die bei 110 ° C/16 h getrocknet und bei 500 ° C/24 h calciniert werden.

Katalysator B

Katalysator B wird hergestellt, indem ein handelsüblicher Na-Y-Zeolith in der Pulverform mit Boehmit (Gewichtsverhältnis 60 : 40) zu 2-mm-Strängen verformt wird, die bei 110 ° C/16 h getrocknet und bei 500 ° C/16 h calciniert werden.

Die Stränge werden mit 20 %iger $NH_4Cl$-Lösung (Massenverhältnis 1 : 15) bei 80 ° C ausgetauscht. Dann wird Chlorid-frei gewaschen, bei 110 ° C getrocknet und 5 h bei 500 ° C calciniert. Der Na-Gehalt beträgt 0,40 %.

Katalysator C

Ein Aluminosilikatzeolith vom Pentasil-Typ wird unter hydrothermalen Bedingungen bei autogenem Druck und 150 ° C aus 650 g hochdispersem $SiO_2$, 203 g $Al_2(SO_4)_3$ x 18 $H_2O$ in 10 kg einer wäßrigen 1,6-Hexandiamin-Lösung (Mischung 50 : 50 Gew.%) in einem Rührautoklaven hergestellt. Nach Abfiltrieren und Auswaschen wird das kristalline Reaktionsprodukt bei 110 ° C/24 h getrocknet und bei 500 ° C/24 h calciniert. Dieser Aluminosilikatzeolith enthält 92,8 Gew.% $SiO_2$ und 4,2 Gew.% $Al_2O_3$. Mit diesem Material werden durch Verformen mit einem Verformungshilfsmittel 2-mm-Stränge hergestellt, die bei 110 ° C/16 h getrocknet und bei 500 ° C/24 h calciniert werden.

Katalysator D

Katalysator D wird erhalten durch Verformung des Aluminosilikatzeolithen (vgl. Katalysator C) mit Boehmit (Gewichtsverhältnis 60 : 40) zu 2-mm-Strängen, die bei 110 °C/16 h getrocknet und bei 500 °C/16 h calciniert werden.

Katalysator E

Für Katalysator E wird ein nach dem bei Katalysator B beschriebenen Verfahren mit $(NH_4)_2SO_4$ ausgetauschtes Na-Y-Zeolithpulver (Na-Gehalt: 0,07 % Na) und ein Borosilikatzeolith (Herstellvorschrift siehe Katalysator A) im Massenverhältnis 1 : 1 gründlich vermischt und unter Zugabe eines Verformungshilfsmittels zu 2-mm-Strängen verformt. Die Strangpreßlinge werden bei 110 °C 16 h getrocknet und 16 h bei 500 °C calciniert.

Beispiel 1

Ein Gemisch aus Imidazol, Essigsäure und Essigsäureanhydrid (Molverhältnis 1:4:1.1) wird verdampft und mit einem Stickstoffstrom von 50 l/h bei 400 bis 450 °C und einer Belastung von 1 $h^{-1}$ über den Katalysator A geleitet.

Das Reaktionsprodukt wird in einer Glasapparatur kondensiert. Der Umsatz ist 57 % und die Selektivität für 4-Acetylimidazol 56 %. Die Ausbeute an 4-Acetylimidazol bezogen auf Imidazol ist 32 % der Theorie.

Beispiel 2

Ein Gemisch aus 2-Methylimidazol, Essigsäure und Essigsäureanhydrid (Molverhältnis = 1 : 4 : 1) wird verdampft und mit einem Stickstoffstrom von 50 l/h bei 400 °C und einer Belastung von 1 $h^{-1}$ über den Katalysator A geleitet.

Das Reaktionsprodukt wird in einer Glasapparatur kondensiert. Es ergibt sich ein Umsatz von 63 % und eine Selektivität für 2-Methyl-4-acetylimidazol von 85 %. Die Ausbeute an 2-Methyl-4-acetylimidazol bezogen auf 2-Methylimidazol ist 54 % der Theorie.

Beispiele 3 - 6

Ein Gemisch aus 4-Methylimidazol, Essigsäureanhydrid und THF im Molverhältnis 1 : 1 : 1 wird verdampft und über die in Tabelle 1 angegebenen Katalysatoren geleitet (siehe Tabelle 1).

Tabelle 1

| Acetylierung von 4-Methylimidazol | | | | | | | | |
|---|---|---|---|---|---|---|---|---|
| Beispiel | Katalysator | Laufzeit [h] | Temp. [°C] | WHSV [$h^{-1}$] | GC-Analyse des Austrags* | | | |
| | | | | | 4-Methylimidazol [%] | N-Acetyl-4-methylimidazol [%] | 5-acetyl-4-methylimidazol [%] | 2-Acetyl-4-methylimidazol [%] |
| 3 | B | 6 | 520 | 4 | 6,8 | 25,0 | 20,4 | 16,3 |
| 3 | B | 12 | 520 | 4 | 7,0 | 19,6 | 19,1 | 20,6 |
| 4 | A | 6 | 520 | 4 | 24,4 | 10,9 | 14,7 | 7,8 |
| 5 | C | 6 | 520 | 4 | 14,2 | 12,4 | 10,9 | 5,2 |
| 6 | D | 6 | 520 | 4 | 17,7 | 13,6 | 14,3 | 10,1 |

* Probe gesammelt über 2 h

Beispiele 7 - 9

2-Methylimidazol wird mit Essigsäure umgesetzt. Das Reaktionsgemisch wird mit THF verdünnt (siehe Tabelle 2).

Tabelle 2

| Acetylierung von 2-Methylimidazol | | | | | | | |
|---|---|---|---|---|---|---|---|
| Beispiel | Katalysator | Laufzeit [h] | Essigsäure: 2-Methylimidazol: THF(molar) | Temp. [°C] | WHSV [$h^{-1}$] | GC-Analyse des Austrags* | |
| | | | | | | 2-Methylimidazol [%] | 4-Acetyl-2-methylimidazol [%] |
| 7 | A | 4 | 2 : 1 : 0 | 450 | 4 | 42,5 | 26,6 |
| 8 | C | 4 | 2 : 1 : 0 | 450 | 4 | 50,1 | 17,6 |
| 9 | E | 4 | 3 : 1 : 1 | 520 | 4 | 38,8 | 9,3 |

* Probe gesammelt über 2 h

Beispiel 10 - 12

Imidazol wird mit Essigsäure im molaren Verhältnis 1 : 2 gemischt und über verschiedenen Katalysatoren umgesetzt (siehe Tabelle 3).

Tabelle 3

| Acetylierung von Imidazol. | | | | | | | |
|---|---|---|---|---|---|---|---|
| Beispiel | Katalysator | Laufzeit [h] | Temp. [°C] | WHSV [$h^{-1}$] | GC-Analyse des Austrags* | | |
| | | | | | Imidazol [%] | N-Acetylimidazol [%] | C-Acetylimidazol [%] |
| 10 | A | 6 | 450 | 3 | 45,3 | 0,1 | 8,1 |
| 11 | B | 6 | 450 | 3 | 48,3 | 0,2 | 6,8 |
| 12 | C | 6 | 450 | 2 | 44,4 | 0,1 | 11,0 |

* Probe gesammelt über 2 h

Beispiele 13 - 14

Pyrazol wird mit einer äquimolaren Menge Essigsäureanhydrid in der Gasphase umgesetzt (siehe Tabelle 4).

Tabelle 4

| Acetylierung von Pyrazol | | | | | | | |
|---|---|---|---|---|---|---|---|
| Beispiel | Katalysator | Laufzeit [h] | Temp. [°C] | WHSV [$h^{-1}$] | GC-Analyse des Austrags* | | |
| | | | | | Pyrazol [%] | N-Acetylpyrazol [%] | C-Acetylpyrazol [%] |
| 13 | C | 2 | 450 | 3 | 35,0 | 24,4 | 3,3 |
| 14 | A | 6 | 400 | 4 | 9,9 | 52,6 | 6,1 |

* Probe gesammelt über 2 h

**Patentansprüche**

**1.** Verfahren zur Herstellung von acylierten Imidazolen und Pyrazolen der Formel I und II

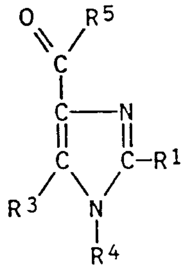
(I)

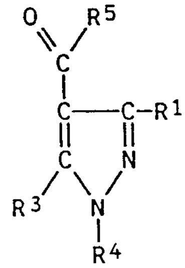
(II)

in denen $R^1$ und $R^3$ gleich oder verschieden sein können und unabhängig voneinander H-, Alkyl- mit 1 bis 12 Kohlenstoffatomen, Alkenyl- mit bis zu 12 Kohlenstoffatomen, Alkoxyl- mit 1 bis 12 Kohlenstoffatomen, Aryl-, Aralkyl- oder Alkylarylreste,
$R^4$ H-, Alkyl- mit 1 bis 12 Kohlenstoffatomen, Alkenyl- mit bis zu 12 Kohlenstoffatomen, Aryl-, Aralkyl- oder Alkylarylreste sowie $R^5$ = Alkyl- mit 1 bis 12 Kohlenstoffatomen, Alkenyl- mit bis zu 12 Kohlenstoffatomen, Aryl-, Aralkyl-, Alkylaryl- oder Carboxyreste bedeuten, dadurch gekennzeichnet, daß man fünfgliedrige Heteroaromaten der Formel III bzw. IV

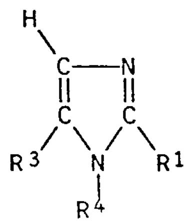 (III) 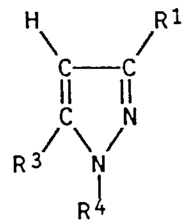 (IV)

in denen $R^1$, $R^3$ und $R^4$ die oben genannten Bedeutungen haben, mit Acylierungsmitteln der Formel V

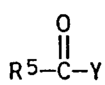 (V)

in der $R^5$ die oben genannte Bedeutung hat und Y einen Halogenid-, Alkoxy-, Acyloxy-, Hydroxy-, Amid- oder Carboxyrest bedeutet, in Gegenwart von Zeolithen als Katalysatoren in der Flüssigphase bei Temperaturen von 50 bis 300 °C oder in der Gasphase bei Temperaturen von 300 bis 600 °C umsetzt.

2. Verfahren nach Anspruch 1, dadurch gekennzeichnet, daß man als Katalysatoren Zeolithe des Pentasil-Typs verwendet.

3. Verfahren nach Anspruch 1, dadurch gekennzeichnet, daß man als Katalysatoren Boro-, Eisen- oder Aluminosilikatzeolithe verwendet.

4. Verfahren nach Anspruch 1, dadurch gekennzeichnet, daß man als Katalysatoren Zeolithe des Faujasit-Typs verwendet.

5. Verfahren nach Anspruch 1 bis 4, dadurch gekennzeichnet, daß man als Katalysatoren mit Alkali-, Erdalkalimetall, seltenen Erdmetallen, Übergangsmetallen und/oder Phosphor dotierte Zeolithe als Katalysatoren verwendet.

6. Verfahren nach Anspruch 5, dadurch gekennzeichnet, daß man als Katalysatoren mit Cäsium dotierte Zeolithe verwendet.

7. Verfahren nach Anspruch 5, dadurch gekennzeichnet, daß man als Katalysatoren mit Ce und/oder La dotierte Zeolithe verwendet.

## Claims

1. A process for the preparation of an acylated imidazole or pyrazole of the formula I or II

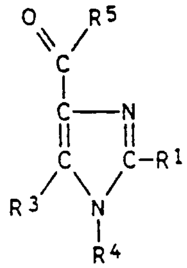 (I) 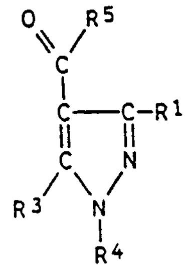 (II)

where $R^1$ and $R^3$ may be identical or different and independently of one another are each H, alkyl of 1 to 12 carbon atoms, alkenyl of up to 12 carbon atoms, alkoxy of 1 to 12 carbon atoms, aryl, aralkyl or alkylaryl,
$R^4$ is H, alkyl of 1 to 12 carbon atoms, alkenyl of up to 12 carbon atoms, aryl, aralkyl or alkylaryl and
$R^5$ is alkyl of 1 to 12 carbon atoms, alkenyl of up to 12 carbon atoms, aryl, aralkyl, alkylaryl or carboxyl,
wherein a five-membered hetaromatic of the formula III or IV

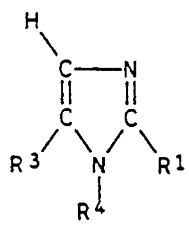 (III) 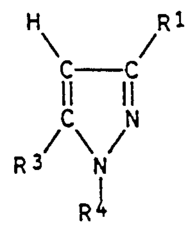 (IV)

where $R^1$, $R^3$ and $R^4$ have the abovementioned meanings, is reacted with an acylating agent of the formula V

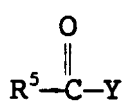 (V)

where $R^5$ has the abovementioned meanings and Y is halide, alkoxy, acyloxy, hydroxyl, amido or carboxyl, in the presence of a zeolite as a catalyst, at 50-300 °C in the liquid phase or at 300-600 °C in the gas phase.

**2.** A process as claimed in claim 1, wherein the catalyst used is a zeolite of the pentasil type.

**3.** A process as claimed in claim 1, wherein the catalyst used is a borosilicate, iron silicate or aluminosilicate zeolite.

**4.** A process as claimed in claim 1, wherein the catalyst used is a zeolite of the faujasite type.

**5.** A process as claimed in any of claims 1 to 4, wherein the catalyst used is a zeolite doped with an alkali metal, alkaline earth metal, rare earth metal, transition metal and/or phosphorus.

**6.** A process as claimed in claim 5, wherein the catalyst used is a cesium-doped zeolite.

**7.** A process as claimed in claim 5, wherein the catalyst used is a zeolite doped with Ce and/or La.

**Revendications**

**1.** Procédé de préparation d'imidazoles et de pyrazoles acylés de formules I et II

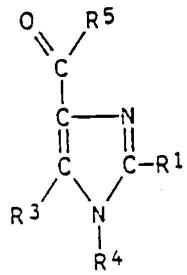 (I) 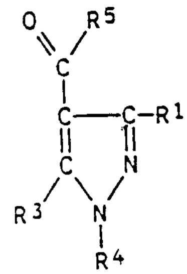 (II)

dans lesquelles $R^1$ et $R^3$ peuvent être identiques ou différents et représentent, indépendamment l'un de l'autre, des atomes d'hydrogène, des restes alkyle à 1-12 atomes de carbone, alcényle contenant jusqu'à 12 atomes de carbone, alcoxy contenant jusqu'à 12 atomes de carbone, aryle, aralkyle ou alkylaryle,
$R^4$ représente un atome d'hydrogène, un reste alkyle à 1-12 atomes de carbone, alcényle contenant jusqu'à 12 atomes de carbone, aryle, aralkyle ou alkylaryle et
$R^5$ représente un reste alkyle à 1-12 atomes de carbone, alcényle contenant jusqu'à 12 atomes de carbone, aryle, aralkyle, alkylaryle ou carboxy,
caractérisé en ce qu'on fait réagir des carbures hétéroaromatiques à cinq chaînons de formules III et IV respectivement

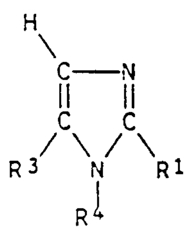 (III) 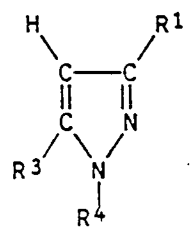 (IV)

dans lesquelles $R^1$, $R^3$ et $R^4$ ont les significations données ci-dessus, avec des agents d'acylation de formule V

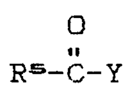 (V)

dans laquelle $R^5$ a la signification donnée ci-dessus et Y représente un reste halogénure, alcoxy, acyloxy, hydroxy, amide ou carboxy, en présence de zéolithes servant de catalyseur, en phase liquide à des températures de 50 à 300 ° C ou en phase gazeuse à des températures de 300 à 600 ° C.

**2.** Procédé selon la revendication 1, caractérisé en ce qu'on utilise, comme catalyseurs, des zéolithes du type pentasil.

**3.** Procédé selon la revendication 1, caractérisé en ce qu'on utilise, comme catalyseurs, des zéolithes de boro-, ferro- ou aluminosilicate.

**4.** Procédé selon la revendication 1, caractérisé en ce qu'on utilise, comme catalyseurs, des zéolithes du type faujasite.

**5.** Procédé selon l'une quelconque des revendications 1 à 4, caractérisé en ce qu'on utilise, comme catalyseurs, des zéolithes dopées avec des métaux alcalins, des métaux alcalino-terreux, des métaux des terres rares, des métaux de transition et/ou du phosphore.

**6.** Procédé selon la revendication 5, caractérisé en ce qu'on utilise, comme catalyseurs, des zéolithes

dopées avec du césium.

7. Procédé selon la revendication 5, caractérisé en ce qu'on utilise, comme catalyseurs, des zéolithes dopées avec Ce et/ou La.